(19) Europäisches Patentamt
European Patent Office
Office européen des brevets

(11) Publication number : 0 334 429 B1

(12) EUROPEAN PATENT SPECIFICATION

(45) Date of publication of patent specification : 19.11.92 Bulletin 92/47

(51) Int. Cl.$^5$ : A61K 31/35, A61K 45/06, C07D 311/58

(21) Application number : 89200661.0

(22) Date of filing : 16.03.89

(54) **Agents for lowering the blood pressure.**

(30) Priority : 23.03.88 US 172747

(43) Date of publication of application : 27.09.89 Bulletin 89/39

(45) Publication of the grant of the patent : 19.11.92 Bulletin 92/47

(84) Designated Contracting States : AT BE CH DE ES FR GB GR IT LI LU NL SE

(56) References cited : EP-A- 0 145 067

(73) Proprietor : JANSSEN PHARMACEUTICA N.V.
Turnhoutsebaan 30
B-2340 Beerse (BE)

(72) Inventor : Xhonneux, Raymond Mathieu
Hei-ende 58
B-2151 Vlimmeren (BE)
Inventor : Van Lommen, Guy Rosalia Eugène
Klets 34
B-2590 Berlaar (BE)

EP 0 334 429 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

In U.S. Pat. No. 4,654,362 there are described 2,2'-iminobisethanol derivatives having β-adrenergic blocking properties. It now has been found that a certain class of isomers of said bisethanol derivatives potentiate the activity of blood pressure reducing agents.

The present invention is concerned with the use for the manufacture of a medicament for potentiating the effects of blood pressure reducing agents having adrenergic and/or vasodilating activity, other than the agents of formula (I) as defined hereinafter, of a compound of formula

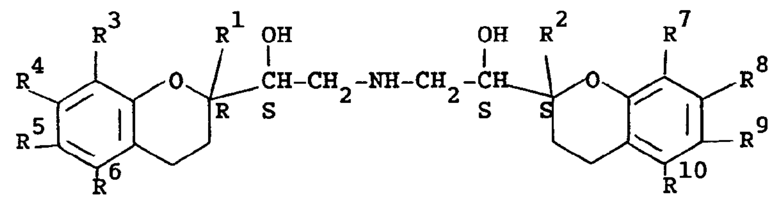

(I),

or the pharmaceutically acceptable acid addition salts thereof, wherein

$R^1$ and $R^2$ each independently are hydrogen or $C_{1-6}$alkyl;

$R^3$, $R^4$, $R^5$, $R^6$, $R^7$, $R^8$, $R^9$ and $R^{10}$ each independently are hydrogen, halo, $C_{1-6}$alkyl, $C_{1-6}$alkyloxy, hydroxy, cyano, carboxy or $C_{1-6}$alkyloxycarbonyl;

or two vicinal radicals of $R^3$, $R^4$, $R^5$, $R^6$, $R^7$, $R^8$, $R^9$ and $R^{10}$ taken together may form a -CH=CH-CH=CH- or -$(CH_2)_4$- radical.

As used in the foregoing definitions the term halo is generic to fluoro, chloro, bromo and iodo; the term "$C_{1-6}$alkyl" defines straight and branch chained saturated hydrocarbon radicals having from 1 to 6 carbon atoms such as, for example, methyl, ethyl, 1-methylethyl, 1,1-dimethylethyl, propyl, 2-methylpropyl, butyl, pentyl and hexyl.

The descriptors R and S as used in the above formula (I) indicate the absolute configuration at the respective carbon atoms. The carbon atom bearing $R^1$ has the R configuration, whereas the carbon atoms bearing the hydroxy functions and the carbon atoms bearing $R^2$ have the S configuration.

Preferred compounds of formula (I) are those wherein $R^3$, $R^4$, $R^6$, $R^7$, $R^8$ and $R^{10}$ are hydrogen.

Particularly preferred are those preferred compounds wherein $R^5$ and $R^9$ are hydrogen or halo, particularly fluoro.

The most preferred compound is [2R,αS,2'S,α'S]-α,α'-[imino-bismethylene]bis[6-fluoro-3,4-dihydro-2H-1-benzopyran-2-methanol] or a pharmaceutically acceptable acid addition salt thereof.

The compounds of formula (I) can be prepared following the procedures described in U.S. Pat. No. 4,654,362. Some particular ways of obtaining the compounds of formula (I) will be described hereinafter in some more detail.

The compounds of formula (I) can be prepared by reacting an oxirane of formula (II-a) or (II-b) with an amine of formula (III-a) or (III-b).

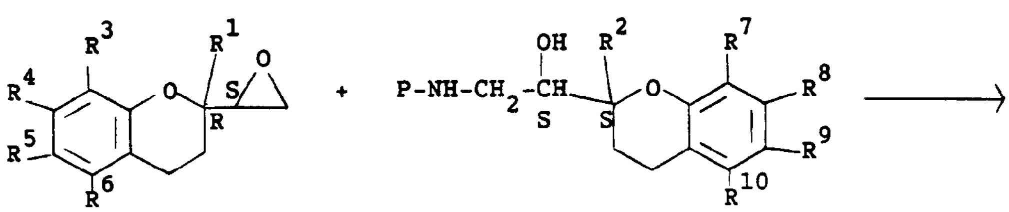

(I)

(II-a) (III-a)

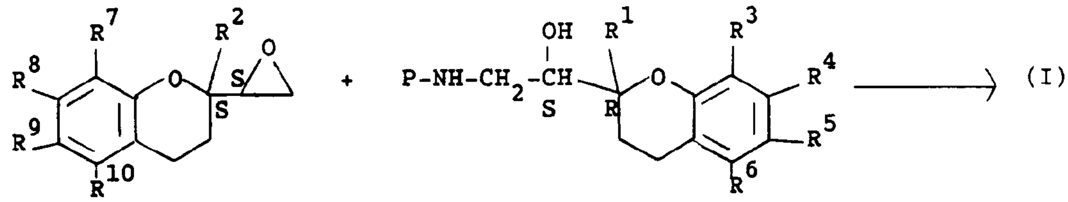


(II-b) (III-b)

In (III-a) and (III-b), P is either hydrogen or an appropriate protecting group, for example an allyl group, or in particular P may be a benzyl group. Or, a reagent P-$NH_2$ may be reacted with (II-a) and (II-b) in a one-pot procedure. The above described reactions to prepare a compound of formula (I) may be conducted in a reaction-inert solvent such as, for example, an aromatic hydrocarbon, e.g. benzene or methylbenzene; an alkanol, e.g. methanol, ethanol, propanol; a ketone, e.g. 2-propanone, 4-methyl-2-pentanone; an ether, e.g. 1,4-dioxane, tetrahydrofuran, 1,1′-oxybisethane; a dipolar aprotic solvent, e.g. N,N-dimethylformamide or N,N-dimethylacetamide. In certain instances, in order to increase the reaction rate, it may be appropriate to heat the reaction mixture.

If in the above reactions P is other than hydrogen, the N-protected derivatives of formula (I) are obtained wherefrom the compounds of formula (I) themselves can be obtained by a deprotection reaction. For example, where P is allyl, by reaction with an appropriate noble metal compound such as $PdCl_2$ or $Rh[P(C_6H_5)_3]Cl$, or where P is benzyl, by a catalytic hydrogenation procedure, e.g. palladium or platinum on charcoal in a suitable solvent such as an ether, e.g. 1,4-dioxane, tetrahydrofuran, an alkanol, e.g. methanol, ethanol, an alkoxyalkanol, e.g. methoxyethanol.

The intermediates of formula (III-a) or (III-b) are obtained by the reaction of the amine P-$NH_2$ with (II-b) or (II-a) or, by reacting a reagent $P_2NH$, for example dibenzylamine, with (II-b) or (II-a) and subsequently selectively removing one of the P-groups, e.g. when P is benzyl by a catalytic hydrogenation procedure using one equivalent hydrogen. The afore described reactions to prepare (III-a) or (III-b) are conducted following the same procedures as described hereinabove for the preparation of the compounds (I).

The starting materials (II-a) are obtained by an oxirane formation reaction from an aldehyde of formula (IV-a), e.g. by reaction of the latter with a trimethylsulfoxonium halide, or from an ethylene of formula (Va) by reaction of the latter with a peroxide, e.g. a haloperbenzoic acid. In the same way, the intermediate (II-b) is obtained from the corresponding S-isomers (IV-b) or (V-b). The oxiranes of formula (IV-a-1) obtained in the aforementioned oxirane-formation reaction are separated in their stereoisomers, e.g. by HPLC or selective crystallization.

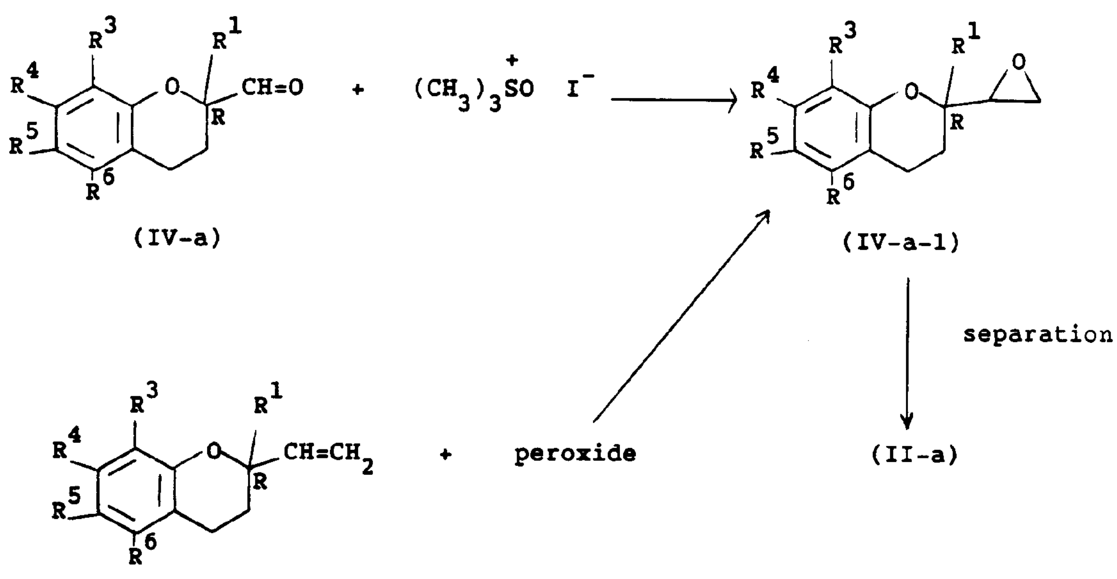


(V-a)

The compounds of formula (IV-a), (IV-b), (V-a) or (V-b) are obtained by a suitable separation procedure, i.e. by HPLC, or by a reduction reaction of the corresponding optically active racemic acids whereas (IV-a) or (IV-b) can be converted to (V-a) or (V-b) by a Wittig reaction. The said corresponding optically active acids in turn can be obtained by conventional separation techniques, i.e. by salt or amide formation with an optically active reagent and a selective crystallization procedure or a HPLC separation.

The compounds of formula (I) have basic properties and, consequently, they may be converted to their therapeutically active non-toxic acid addition salt forms by treatment with appropriate acids, such as, for example, inorganic acids, such as hydrohalic acid, e.g. hydrochloric and hydrobromic, and sulfuric acid, nitric acid and phosphoric acid; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic and 4-amino-2-hydroxybenzoic.

Conversely, the salt form can be converted by treatment with alkali into the free base form.

The compounds of formula (I) with the exception of (RSSS)-α,α′-[iminobis(methylene)bis(3,4-dihydro-2H-1-benzopyran-2-methanol] ethanedioate(1:1) are deemed to be novel compounds and constitute in an additional feature to the present invention.

The compounds of formula (I) and the pharmaceutically acceptable acid addition salts thereof potentiate the activity of blood pressure reducing agents. In particular they potentiate the reduction of the blood pressure and of the heart rate.

As blood pressure reducing agents of which the activity is potentiated there may be mentioned agents having adrenergic and/or vasodilating activity. In particular such agents may be the compounds mentioned in U.S. Pat. Nos. 3,663,607 and 3,836,671, in particular atenolol; U.S. Pat. Nos. 3,337,628 and 3,520,919, in particular propranolol; U.S. Pat. No. 3,873,600, in particular metoprolol; U.S. Pat. No. 3,511,836, in particular prazosin; U.S.Pat. No. 2,484,029, in particular hydralazine; U.S. Pat. No. 2,928,829 in particular guanethidine; U.S. Pat. No. 2,503,059, in particular phentolamine; U.S. Pat. No. 3,261,859, in particular verapamil; U.S. Pat. No. 3,485,847 in particular nifedipine; U.S. Pat. No. 3,910,924, in particular carteolol; German Pat. Nos. 2,458,624 and 2,458,625, in particular celiprolol. A particular group of blood pressure reducing compounds are the compounds of U.S. Pat. No. 4,654,362 other than the compounds of formula (I) and in particular the enantiomers of the compounds of formula (I), i.e. the SRRR-isomers. A particular compound is [2S,αR,2′R,α′R]-α,α′-[iminobismethylene]bis[6-fluoro-3,4-dihydro-2H-1-benzopyran-2-methanol. These groups of active ingredients are listed with the purpose of providing representative examples. The said SRRR isomers and the said particular compound can be prepared following the same procedures as previously described for the preparation of the compounds of formula (I), but starting from the enantiomers of the intermediates (II-a), (III-a), (II-b) and (III-b). The latter enantiomers in turn can be obtained as described hereinabove for the preparation of (II-a), (III-a), (II-b) and (III-b), but starting from the enantiomers of (IV-a) or (V-a) and isolating the appropriate stereoisomers in stereochemical separation procedures. The enantiomers of (IV-a) and (V-a) in the same way can be obtained as described for the preparation of (IV-a) and (V-a) starting from the appropriate enantiomeric starting materials and/or isolating the appropriate stereoisomers in stereochemical separations.

The compounds of formula (I) and the acid addition salts thereof may be administered before, during or after the administration of the blood pressure reducing agent provided that the time of the administration of the compounds of formula (I) in relation to the administration of the blood pressure reducing agent allows the compound of formula (I) to be effective in potentiating the effects of the blood pressure reducing agent. Preferably the compound of formula (I) and the blood pressure reducing agent are administered in the form of suitable compositions. Said compositions are meant to also comprise products containing a compound of formula (I) as defined hereinabove and a blood-pressure reducing agent as a combined preparation for simultaneous, separate or sequential use in blood-pressure reducing therapy. Such products may for example comprise a kit comprising a container with a suitable composition containing a compound of formula (I) and another container containing a composition with a blood pressure reducing agent. Such product may have the advantage that the physician wishing to administer blood pressure reducing therapy can select, based on the diagnosis of the patient to be treated, the appropriate amounts of both components and the sequence of administration.

When administered during the administration of the blood pressure reducing agent, a composition containing both the blood pressure reducing agent and the active ingredient of formula (I) may particularly be convenient.

In a further aspect of the present invention there is provided a composition comprising an amount capable of potentiating the effects of blood pressure reducing agents of a compound of formula (I) as defined hereinabove and a blood pressure reducing agent. In the said composition, the molar ratio between the compound of formula (I) and the blood pressure reducing agent may be other than 1:1, but in particular may be 1:1. The

amount of the active ingredient of formula (I) in such composition will be so that a potentiating effect on the effects of the blood-pressure reducing agent is obtained; the amount of the blood pressure reducing agent will be so that when potentiated, a blood pressure reducing effect is obtained upon administration. In particular, it is contemplated that the molar ratio of the compound of formula (I) to the blood pressure reducing compound may be situated between 50:1 and 1:50, in particular between 20:1 and 1:20, or between 10:1 and 1:10, or between 5:1 and 1:5, more particularly between 2:1 and 1:2. Particular such compositions are those wherein the blood pressure reducing agent is one of the agents pertaining to the patents cited hereinabove, and more particularly the agents specifically mentioned hereinabove.

The present invention also provides a composition comprising a pharmaceutically acceptable carrier and as active ingredient an amount capable of potentiating the effects of blood pressure reducing agents of a novel compound of formula (I) or a pharmaceutically acceptable acid-addition salt thereof, as defined hereinabove.

To prepare such pharmaceutical compositions, an effective amount of the particular compound or compounds, in base or acid-addition salt form, as the active ingredient or active ingredients is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils and alcohols in the case of oral liquid preparations such as suspensions, syrups, elixirs and solutions; or solid carriers such as starches, sugars, kaolin, lubricants, binders and disintegrating agents in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers and suspending agents may be employed. In the compositions suitable for percutaneous administration, the carrier optionally comprises a penetration enhancing agent and/or a suitable wetting agent, optionally combined with suitable additives of any nature in minor proportions, which additives do not cause a significant deletorious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls and tablespoonfuls, and segregated multiples thereof.

Those of skill in treating subjects suffering from an increased blood pressure could easily determine the effective amount from the test results presented hereinafter. In general it is contemplated that an effective daily dose of the compounds of formula (I) or their pharmaceutically acceptable acid-addition salts would be from 0.01 mg/kg to 50 mg/kg body weight, in particular from 0.1 mg/kg to 10 mg/kg body weight and preferably from 0.1 mg/kg to 1 mg/kg body weight.

The following examples are intended to illustrate the scope of the present invention in all its aspects. Unless otherwise stated all parts therein are by weight.

Whenever used in the following examples "A" refers to the isomer which was first isolated and "B" to the one which was subsequently isolated.

## EXPERIMENTAL PART

### A. Preparation of the intermediates

#### Example 1

a) A mixture of 63.4 parts of 6-fluoro-4-oxo-4*H*-1-benzopyran-2-carboxylic acid and 400 parts of acetic acid was hydrogenated at normal pressure and at room temperature with 3 parts of palladium-on-charcoal catalyst 10%. After the calculated amount of hydrogen was taken up, the catalyst was filtered off and the

filtrate was evaporated. The residue was stirred in petroleumether. The product was filtered off and dried in vacuo at 70°C, yielding 49 parts (83%) of 6-fluoro-3,4-dihydro-2H-1-benzopyran-2-carboxylic acid (int. 1).

b) To a stirred solution of 9.75 parts of intermediate 1 in 90 parts of methylbenzene were added 16 parts of thionyl chloride. The mixture was stirred for 2 hours at 60°C. The reaction mixture was evaporated. The residue was taken up twice in 45 parts of methylbenzene and the latter was evaporated each time. The residue was taken up in 90 parts of methylbenzene. There were added first 10.5 parts of N,N-diethylethanamine and then a solution of 14.25 parts of (+)-1,2,3,4,4a,9,10,10a-octahydro-1,4a-dimethyl-7-(1-methylethyl)-1-phenanthrenemethanamine [(+)-dehydroabiethylamine] in 45 parts of methylbenzene. After stirring for 2 hours, the organic layer was washed successively with water, a sodium hydroxide solution 10%, a hydrochloric acid solution 10% and water, dried, filtered and evaporated. The residue was taken up in 120 parts of warm ethanol. The product was filtered off and crystallized from ethanol, yielding 6.6 parts (28.4%) of (A)-6-fluoro-3,4-dihydro-N-[dehydroabiethyl]-2H-1-benzopyran-2-carboxamide (int. 2).

c) A mixture of 6.8 parts of intermediate 2, 75 parts of acetic acid and 36 parts of concentrated hydrochloric acid was stirred for 24 hours at reflux temperature. After cooling, the reaction mixture was poured into water. The product was extracted with 1,1′-oxybisethane. The extract was washed twice with water, dried, filtered and evaporated. The residue was taken up in 1,1′-oxybisethane. 5 Parts of a sodium hydroxide solution were added. The product was filtered off, taken up in trichloromethane and treated with 50 parts of a hydrochloric acid solution 10%. The organic layer was dried, filtered and evaporated, yielding 1.1 parts of (+)-(S)-6-fluoro-3,4-dihydro-2H-1-benzopyran-2-carboxylic acid; mp. 99.7°C $[\alpha]_D^{25}$ =+14.88°(c= 1% in DMF) (int. 3).

d) To a stirred solution of 22.5 parts of intermediate 3 in 180 parts of tetrahydrofuran were added 18.7 parts of 1,1′-carbonylbis[1H-imidazole]. The whole was stirred for 1 hour at room temperature and cooled to -70°C. 136 Parts of a 25% solution of [bis(2-methylpropyl)]aluminum hydride in methylbenzene were added dropwise during a period of 20 minutes. Upon completion, stirring was continued for 20 minutes at -70°C. 40 Parts of methanol were added and the mixture was poured into water. The product was extracted with 1,1′-oxybisethane. The extract was washed successively with a hydrochloric acid solution 10%, water and a sodium hydrogen carbonate solution, dried, filtered and evaporated, yielding 12 parts (57.9%) of (+)-(S)-6-fluoro-3,4-dihydro-2H-1-benzopyran-2-carboxaldehyde as an oily residue (int. 4).

e) 6.3 Parts of a sodium hydride dispersion 50% were washed twice with petroleum ether and then taken up in 250 parts of dimethyl sulfoxide. 29 Parts of trimethylsulfoxonium iodide were added during a period of 30 minutes and stirring was continued for 20 minutes. A solution of 12 parts of intermediate 4 in 10 parts of dimethyl sulfoxide was added dropwise and upon completion, the mixture was stirred for 30 minutes. The reaction mixture was poured into water and the product was extracted with 1,1′-oxybisethane. The extract was washed three times with water, dried, filtered and evaporated. The residue was purified by column chromatography (HPLC) over silica gel using a mixture of methylbenzene and ethyl acetate (90:10 by volume) as eluent. The pure fractions were collected and the eluent was evaporated, yielding 2.1 parts (9.8%) of (+)-[S(S)]-6-fluoro-3,4-dihydro-2-oxiranyl-2H-1-benzopyran as an oily residue (int. 5).

Example 2

a) In the procedure described hereinabove in example 1b) 6.1 parts (26.3%) of the compound (B)-6-fluoro-3,4-dihydro-N-[dehydroabiethyl]-2H-1-benzopyran-2-carboxamide (int. 6) was obtained as a residue.

b) A mixture of 6.1 parts of intermediate 6, 75 parts of acetic acid and 36 parts of concentrated hydrochloric acid was stirred for 24 hours at reflux temperature. The reaction mixture was poured into water. The product was extracted with 1,1′-oxybisethane. The extract was washed twice with water, dried, filtered and evaporated in vacuo. The residue was crystallized from petroleum ether. The product was filtered off and dried, yielding 0.9 parts of (-)-(R)-6-fluoro-3,4-dihydro-2H-1-benzopyran-2-carboxylic acid; mp. 102.5°C $[\alpha]_D^{25}$ = -13.39° (c= 1% in DMF) (int. 7).

c) To a stirred and refluxed solution of 36 parts of intermediate 7 in 400 parts of methanol were added 1.8 parts of sulfuric acid. The mixture was further and refluxed for 4 hours. After cooling, the reaction mixture was evaporated. The residue was taken up in 1,1′-oxybisethane. The mixture was washed successivily twice with a sodium hydrogen carbonate solution and once with water, dried, filtered and evaporated, yielding 33 parts (82.6%) of (-)-(R)-methyl 6-fluoro-3,4-dihydro-2H-1-benzopyran-2-carboxylate as an oily residue (int. 8).

d) To a stirred and cooled (-80°C) solution of 33 parts of intermediate 8 in 450 parts of methylbenzene were added dropwise 255 parts of a solution of [bis(2-methylpropyl)]aluminium hydride in methylbenzene

under nitrogen atmosphere. Stirring was continued for 30 minutes at -80°C. 16 Parts of methanol were added and the reaction mixture was poured into water. The mixture was acidified with hydrochloric acid and the two layers were separated. The organic phase was dried, filtered and evaporated, yielding an oily residue of 32 parts (the residue was set aside). 9.6 Parts of a sodium hydride dispersion 50% were washed first three times with petroleumether and then taken up in 500 parts of dimethyl sulfoxide. 44 parts of trimethylsulfoxonium iodide were added portionwise and after complete addition, the whole was stirred for 20 minutes at room temperature. To the thus obtained mixture was added dropwise a solution of 32 parts of the oily residue, which was set aside (see above), in 20 parts of dimethyl sulfoxide. Upon completion, stirring was continued for 20 minutes at room temperature. The whole was poured into water and the product was extracted with 2,2′-oxybispropane. The extract was dried, filtered and evaporated. The residue was separated by column chromatography (HPLC) over silica gel using a mixture of hexane and ethyl acetate (80:20 by volume) as eluent. The desired fractions were collected and the eluent was evaporated, yielding 8.2 parts (24.8%) of (-)-[R(S)]-6-fluoro-3,4-dihydro-2-oxiranyl-2H-1-benzopyran as a residue (int. 9).

e) A solution of 8.2 parts of intermediate 9 and 20 parts of benzenemethanamine in 80 parts of methanol was stirred overnight at room temperature. The reaction mixture was evaporated and the residue was taken up in 2,2′-oxybispropane. The precipitated product was filtered off and crystallized from acetonitrile. The product was filtered off and dried, yielding 4.6 parts (38.1%) of (-)-[R(S)]-6-fluoro-3,4-dihydro-α-[[(phenylmethyl)amino]methyl]-2H-1-benzopyran-2-methanol (int. 10).

## B. Preparation of the final compounds

### Example 3

a) A solution of 1.8 parts of intermediate 5 and 2 parts of intermediate 10 in 40 parts of ethanol was stirred for 4 hours at reflux temperature. The reaction mixture was evaporated, yielding 3.5 parts (100%) of [2R,αS,2′S,α′S]-α,α′-[[(phenylmethyl)imino]bismethylene]bis-[6-fluoro-3,4-dihydro-2H-1-benzopyran-2-methanol] as a residue (int. 11).

b) A mixture of 3.5 parts of intermediate 11 and 250 parts of 2-methoxyethanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was taken up in trichloromethane and purified by column chromatography over silica gel using trichloromethane as eluent. The pure fractions were collected and the eluent was evaporated. The residue was crystallized twice from acetonitrile. The product was filtered off and dried, yielding 1.2 parts (42%) of [2R,αS,2′S,α′S]-α,α′-[iminobismethylene]bis[6-fluoro-3,4-dihydro-2H-1-benzopyran-2-methanol]; mp. 142.7°C (compound 1).

### Example 4

A mixture of 19.4 parts of (RS,SS)-α,α′-[[(phenylmethyl)imino]bis-(methylene)bis[3,4-dihydro-2H-1-benzopyran-2-methanol], prepared as described in US-4,654,362 (see compound 16 in the experimental part of the latter; the designation "$A^-B^+$ referring to the RSSS isomer) and 243 parts of 2-methoxyethanol was hydrogenated at normal pressure and at room temperature with 2 parts of palladium-on-charcoal catalyst 10%. After the calculated amount of hydrogen was taken up, the reaction mixture was filtered over diatomaceous earth and evaporated. The residue was crystallized twice from acetonitrile, yielding 6.8 parts (43.8%) of (RS,SS)-α,α′-[iminobis(methylene)]]bis[3,4-dihydro-2H-1-benzopyran-2-methanol]; mp. 136.1°C (compound 2).

### Example 5

A mixture of 6 parts of intermediate 10, 5 parts of (SS)-3,4-dihydro-2-oxiranyl-2H-1-benzopyran, prepared as described in example 17 of US-4,654,362 (intermediate 53, the designation "$B^{+}$" referring to the SS-isomer) and 119 parts of ethanol was refluxed for 18 hours. The reaction mixture was evaporated and the residue was added to 275 parts of 2-methoxyethanol and hydrogenated at normal pressure and at room temperature with 2 parts of palladium-on-charcoal catalyst 10%. After the calculated amount of hydrogen was taken up, the catalyst was filtered off and the filtrate was evaporated. The residue was crystallized from acetonitrile, yielding 3.8 parts (49.3%) of (RSSS)-α-[[[2-(3,4-dihydro-2H-1-benzopyran-2-yl)-2-hydroxyethyl]amino]methyl]-6-fluoro-3,4-dihydro-2H-1-benzopyran-2-methanol; mp. 154.2°C (compound 3).

Example 6

Following the same procedures as described in example 5 and starting from (SS)-6-fluoro-3,4-dihydro-α-[[(phenylmethyl)amino]methyl]-2H-1-benzopyran-2-methanol (obtained from the reaction of intermediate 5 with benzenemethanamine) and (SR)-3,4-dihydro-2-oxiranyl-2H-1-benzopyran (obtained as described in example 17, compound 52 of US-4,654,362; the designation "A-" referring to the SR isomer) there was also prepared (SSSR)-α-[[[2(3,4-dihydro-2H-1-benzopyran-2-yl)-2-hydroxyethyl]amino]-methyl]-6-fluoro-3,4-dihydro-2H-1-benzopyran-2-methanol; mp. 140.7°C (compound 4).

C. Pharmacological examples

Adult spontaneous hypertensive rats (6 months of age) were anesthetized by ether inhalation. The femoral artery was dissected and cannulated, and the catheter was connected to a strain-gauge blood pressure transducer. When the animals were fully awake, they were restrained and the systolic and diastolic arterial blood pressure were continuously recorded. An observation period of at least 30 min preceded the administration of the test compound. All test compounds were dissolved in 20% polypropylene glycol and injected intraperitoneally. After administration of the test drug the systolic and diastolic arterial blood pressure and the heart rate were recorded during a period of 120 minutes. The average blood pressure and heart rate was calculated from the results obtained at various time intervals after administration of the test drug. The following table illustrates the difference between treated and untreated animals expressed as a percentage (Δ%) in the systolic and diastolic blood pressure and the heart rate.

**Δ% Changes (average 120 min) in systolic and diastolic (SBP, DBP) and in heart rate (HR) in spontaneous hypertensive rats**

| | * 1.25 mpk | Hydralazine 0.63 mpk | Guanethidine 2.5 mpk | Phentolamine 0.63 mpk |
|---|---|---|---|---|
| SBP | 0 | -7.5 | -9.3 | -9.85 |
| DBP | +2.1 | -9.9 | -6.2 | -13.1 |
| HR | 0.5 | -1.45 | -7.9 | +5.1 |
| | | Hydralazine 0.63 mpk + * 1.25 | Guanethidine 2.5 mpk + * 1.25 | Phentolamine 0.63 mpk + * 1.25 |
| SBP | | -20.9 | -15.7 | -16.7 |
| DBP | | -28 | -16.7 | -21.2 |
| HR | | -3.6 | -17.6 | +0.9 |

| | * 2.5 mpk | Atenolol 10 mpk | Propranolol 5 mpk | Metoprolol 10 mpk | Prazosin 0.01 mpk |
|---|---|---|---|---|---|
| SBP | -7 | -3.7 | -2 | -1.2 | -10.9 |
| DBP | 0 | +5.9 | +12.4 | +12.8 | -11.3 |
| HR | 0 | -28.1 | -20.7 | -16.6 | +1.6 |
| | | Atenolol 10 mpk + * 2.5 | Propranolol 5 mpk + * 2.5 | Metoprolol 10 mpk + * 2.5 | Prazosin 0.01 mpk + * 2.5 |
| SBP | | -21 | -9.6 | -12.7 | -27.6 |
| DBP | | -21 | +3.2 | -4 | -28.7 |
| HR | | -32 | -33.1 | -28.25 | -6.8 |

* = [2R,αS,2'S,α'S]-α,α'-[iminobismethylene]bis[6-fluoro-3,4-dihydro-2<u>H</u>-1-benzopyran-2-methanol]. (compound 1).

## Claims

1. The use for the manufacture of a medicament for potentiating the effects of blood pressure reducing agents having adrenergic and/or vasodilating activity, other than the agents of formula (I) as defined hereinafter, of a compound of formula

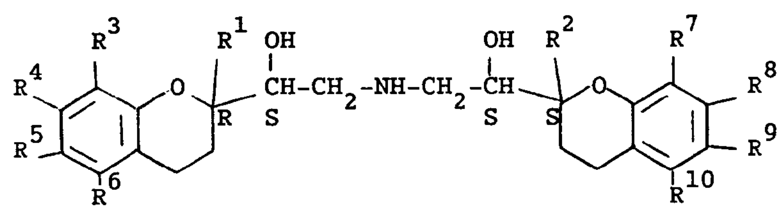

(I),

or a pharmaceutically acceptable acid addition thereof, wherein
$R^1$ and $R^2$ each independently are hydrogen or $C_{1-6}$alkyl;
$R^3$, $R^4$, $R^5$, $R^6$, $R^7$, $R^8$, $R^9$ and $R^{10}$ each independently are hydrogen, halo, $C_{1-6}$alkyl, $C_{1-6}$alkyloxy, hydroxy, cyano, carboxy or $C_{1-6}$alkyloxycarbonyl;
or two vicinal radicals of $R^3$, $R^4$, $R^5$, $R^6$, $R^7$, $R^8$, $R^9$ and $R^{10}$ taken together may form a -CH=CH-CH=CH- or $-(CH_2)_4-$ radical.

2. The use according to claim 1 wherein $R^3$, $R^4$, $R^6$, $R^7$, $R^8$ and $R^{10}$ are hydrogen.

3. The use according to claim 1 wherein the compound is [2R,αS,-2'S,α'S]-α,α'-[iminobismethylene]bis[6-fluoro-3,4-dihydro-2<u>H</u>-1-benzopyran-2-methanol].

4. A pharmaceutical composition comprising a pharmaceutically acceptable carrier, a compound of formula

(I) as defined in any of claims 1 to 3, and a blood pressure reducing agent having adrenergic and/or vasodilating activity, said agent being other than the said compound of formula (I).

5. A composition according to claim 4 wherein the blood pressure reducing agent is selected from atenolol, propranolol, metoprolol, prazosin, hydralazine, guanethidine, phentolamine, verapamil, nifedipine, carteolol and celiprolol.

6. A composition according to claim 4 wherein the blood pressure reducing agent is [2S,αR,2'R,α'R)-α,α'-[iminobismethylene]bis-[6-fluoro-3,4-dihydro-2H-1-benzopyran-2-methanol].

7. A composition according to claim 5 or 6 wherein the molar ratio of both active ingredients is 1:1.

8. A composition according to claim 5 or 6 wherein the molar ratio of both active ingredients is other than 1:1.

9. A product containing a chemical compound of formula (I) as defined in any of claims 1 to 3, and a blood pressure reducing agent, as a combined preparation for simultaneous, separate or sequential use in blood pressure reducing therapy.

10. A chemical compound of formula (I) as defined in any of claims 1 to 3, the compound (RSSS)-α,α'-[iminobis(methylene)bis-(3,4-dihydro-2H-1-benzopyran-2-methanol] ethanedioate(1:1) being excluded.

11. A compound according to claim 10 for use as a medicine.

12. A pharmaceutical composition comprising a pharmaceutically acceptable carrier and as active ingredient an amount capable of potentiating the effects of blood pressure reducing agents of a compound of formula (I) as claimed in claim 10.

13. A process for preparing a composition as claimed in claims 4 to 8 and in claim 12, characterized in that the active ingredient or active ingredients is or are intimately mixed with the pharmaceutical carrier.

14. A process for preparing a compound as claimed in claim 10,
characterized by
reacting an oxirane of formula

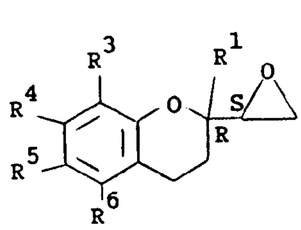

(II-a)

or

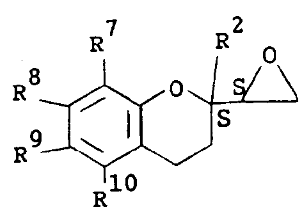

(II-b)

with an amine of formula

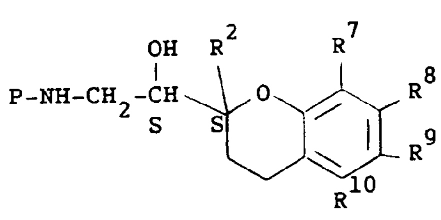

(III-a)

respectively of formula

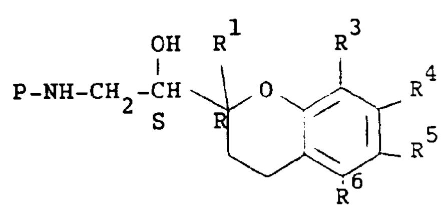

(III-b)

or reacting a reagent $PNH_2$ with (II-a) and (II-b) in a one-pot procedure; in a reaction inert solvent; and

wherein P is hydrogen or a N-protective group; and where P is a N-protective group, deprotecting the thus obtained N-protected derivatives of formula (I); and, if desired, preparing a pharmaceutically acceptable acid addition salt by treatment with an acid; or conversely, preparing the free base form by treatment with a base.

## Patentansprüche

1. Verwendung einer Verbindung der Formel

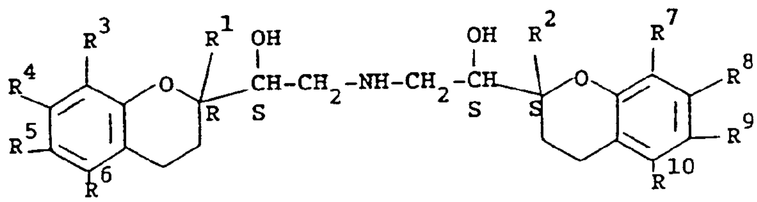

(I)

oder eines pharmazeutisch annehmbaren Säureadditionssalzes hievon, worin
$R^1$ und $R^2$ jeweils unabhängig Wasserstoff oder $C_{1-6}$-Alkyl bedeuten;
$R^3$, $R^4$, $R^5$, $R^6$, $R^7$, $R^8$, $R^9$ und $R^{10}$ jeweils unabhängig Wasserstoff, Halogen, $C_{1-6}$-Alkyl, $C_{1-6}$-Alkyloxy, hydroxy, Cyano, Carboxy oder $C_{1-6}$-Alkyloxycarbonyl bedeuten;
oder zwei benachbarte Reste von $R^3$, $R^4$, $R^5$, $R^6$, $R^7$, $R^8$, $R^9$ und $R^{10}$ gemeinsam einen -CH=CH-CH=CH- oder $-(CH_2)_4$-Rest bilden können,
zur Herstellung eines Medikaments zum Potenzieren der Wirkungen von blutdrucksenkenden Mitteln mit adrenerger und/oder vasodilatorischer Aktivität, die von den Mitteln der Formel (I) gemäß vorstehender Definition verschieden sind.

2. Verwendung nach Anspruch 1, worin $R^3$, $R^4$, $R^6$, $R^7$, $R^8$ und $R^{10}$ Wasserstoff bedeuten.

3. Verwendung nach Anspruch 1, worin die Verbindung [2R,αS,2'S, α'S]-α, α'-[Iminobismethylen]bis[6-fluor-3,4-dihydro-2H-1-benzopyran-2-methanol] ist.

4. Pharmazeutische Zusammensetzung, umfassend einen pharmazeutisch annehmbaren Träger, eine Verbindung der Formel (I), wie in einem der Ansprüche 1 bis 3 definiert, und ein blutdrucksenkendes Mittel mit adrenerger und/oder vasodilatorischer Aktivität, welches Mittel von der genannten Verbindung der Formel (I) verschieden ist.

5. Zusammensetzung nach Anspruch 4, worin das blutdrucksenkende Mittel unter Atenolol, Propranolol, Metoprolol, Prazosin, Hydralazin, Guanethidin, Phentolamin, Verapamil, Nifedipin, Carteolol und Celiprolol ausgewählt ist.

6. Zusammensetzung nach Anspruch 4, worin das blutdrucksenkende Mittel [2S, αR,2'R, α 'R]-α,α'-[Iminobismethylen]bis[6-fluor-3,4-dihydro-2H-1-benzopyran-2-methanol] ist.

7. Zusammensetzung nach Anspruch 5 oder 6, worin das Molverhältnis der beiden wirksamen Bestandteile 1:1 beträgt.

8. Zusammensetzung nach Anspruch 5 oder 6, worin das Molverhältnis der beiden wirksamen Bestandteile von 1:1 verschieden ist.

9. Produkt, das eine chemische Verbindung der Formel (I), wie in einem der Ansprüche 1 bis 3 definiert, und ein blutdrucksenkendes Mittel enthält, als ein kombiniertes Präparat für die gleichzeitige, getrennte oder aufeinanderfolgende Anwendung in der Blutdrucksenkungstherapie.

10. Chemische Verbindung der Formal (I), wie in einem der Ansprüche 1 bis 3 definiert, unter Ausnahme der Verbindung (RSSS)-α, α'-[Iminobis-(methylen)bis(3,4-dihydro-2H-1-benzopyran-2-methanol]-ethandioat(1:1).

11. Verbindung nach Anspruch 10 zur Verwendung als eine Medizin.

12. Pharmazeutische Zusammensetzung, umfassend einen pharmazeutisch annehmbaren Träger und als wirksamen Bestandteil eine zum Potenzieren der Wirkungen von blutdrucksenkenden Mitteln fähige Menge einer Verbindung der Formel (I), wie in Anspruch 10 beansprucht.

13. Verfahren zur Herstellung einer Zusammensetzung nach den Ansprüchen 4 bis 8 und 12, dadurch gekennzeichnet, daß der wirksame Bestandteil oder die wirksamen Bestandteile innig mit dem pharmazeutischen Träger vermischt wird oder werden.

14. Verfahren zur Herstellung einer Verbindung nach Anspruch 10, gekennzeichnet durch Umsetzen eines Oxirans der Formel

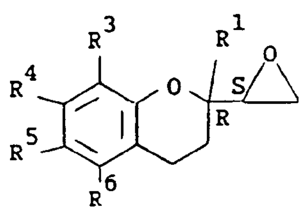

oder

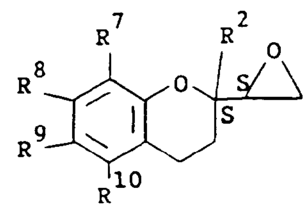

(II-a) (II-b)

mit einem Amin der Formel

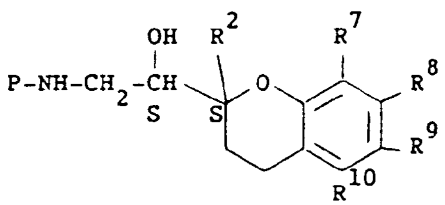

bzw.

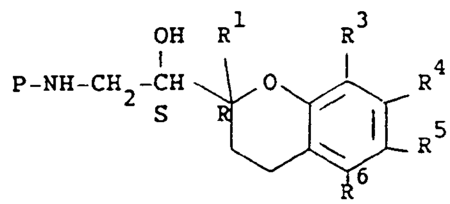

(III-a) (III-b)

oder Umsetzen eines Reagens $PNH_2$ mit (II-a) und (II-b) in einem Eintopfverfahren in einem reaktionsinerten Lösungsmittel, wobei P für Wasserstoff oder eine N-Schutzgruppe steht; und, falls P eine N-Schutzgruppe bedeutet, Abspalten der Schutzgruppe aus den solcherart erhaltenen N-geschützten Derivaten der Formel (I);
und gewünschtenfalls Herstellen eines pharmazeutisch annehmbaren Säureadditionssalzes durch Behandlung mit einer Säure; oder umgekehrt, Herstellung der freien Basenform durch Behandlung mit einer Base.

## Revendications

1. L'utilisation pour la fabrication d'un médicament destiné à augmenter les effets des agents abaissant la pression sanguine, qui présentent une activité adrénergique et/ou vasodilatatrice, autres que les agents de formule (I) tels que définis ci-après, d'un composé de formule

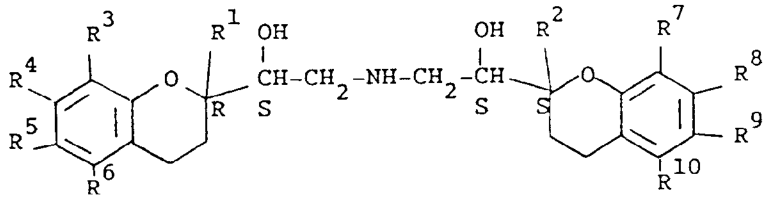

(I)

ou d'un de ses sels d'addition pharmaceutiquement acceptables, dans laquelle $R^1$ et $R^2$ représentent chacun, indépendamment, de l'hydrogène ou un alkyle en $C_1$ à $C_6$;

$R^3$, $R^4$, $R^5$, $R^6$, $R^7$, $R^8$, $R^9$ et $R^{10}$ représentent chacun indépendamment, un radical hydrogène, halo, alkyle en $C_1$ à $C_6$, alkyloxy en $C_1$ à $C_6$, hydroxy, cyano, carboxy ou alkyloxycarbonyle en $C_1$ à $C_6$ ;

ou deux radicaux voisins parmi $R^3$, $R^4$, $R^5$, $R^6$, $R^7$, $R^8$, $R^9$ et $R^{10}$ pris conjointement peuvent former un radical -CH=CH-CH=CH- ou $-(CH_2)_4-$.

2. L'utilisation selon la revendication 1, dans laquelle $R^3$, $R^4$,$R^6$, $R^7$, $R^8$ et $R^{10}$ sont de l'hydrogène.

3. L'utilisation selon la revendication 1, dans laquelle le composé est le (2R, α S,-2'S,α'S]-α,α'-(iminobisméthylène]bis(6-fluoro-3,4-dihydro-2H-1-benzopyran-2-méthanol].

4. Une composition pharmaceutique comportant un support pharmaceutiquement acceptable, un composé de formule (I) tel que défini dans l'une quelconque des revendications 1 à 3, et un agent d'abaissement de la pression sanguine, présentant une activité adrénergique et/ou vasodilatatrice, ledit agent étant différent dudit composé de formule (I).

5. Une composition selon la revendication 4, dans laquelle l'agent abaissant la pression sanguine est choisi parmi l'aténolol, le propanolol,le métoprolol, la prazosine, l'hydralazine, la guanethidine, la phentolamine, le vérapamil, la nifédipine, le cartéolol, et le céliprolol.

6. Une composition selon la revendication 4 dans laquelle l'agent abaissant la pression sanguine est le [2S,αR, 2'R,α'R)-α,α'-(iminobisméthylène]bis-(6-fluoro-3,4-dihydro-2H-1-benzopyran-2-méthanol].

7. Une composition selon la revendication 5 ou 6 selon laquelle le rapport molaire entre les deux ingrédients actifs est de 1:1.

8. Une composition selon la revendication 5 ou 6, dans laquelle le rapport molaire entre les deux ingrédients actifs est différent de 1:1.

9. Un produit renfermant un composé chimique de formule (I) tel que défini dans l'une quelconque des revendications 1 à 3, et un agent abaissant la pression sanguine, sous la forme d'une préparation combinée pour une utilisation simultanée, séparée ou séquentielle dans une thérapie d'abaissement de la pression sanguine.

10. Un composé chimique de formule (I) tel que défini dans l'une quelconque des revendications 1 à 3, le composé (RSSS)-α,α'-(iminobis(méthylène)bis-(3,4-dihydro-2H-1-benzopyran-2-méthanol] éthanedioate (1:1) étant exclus.

11. Un composé selon la revendication 10, destiné à être utilisé comme médicament.

12. Une composition pharmaceutique comportant un support pharmaceutiquement acceptable et, comme ingrédient actif, une quantité susceptible d'augmenter les effets des agents abaissant la pression sanguine d'un composé de formule (I) tel que revendiqué dans la revendication 10.

13. Un procédé pour préparer une composition tel que revendiqué dans les revendications 4 à 8 et dans la revendication 12, caractérisé en ce que l'ingrédient actif ou les ingrédients actifs est ou sont intimement mélangés avec le support pharmaceutique.

14. Un procédé pour préparer un composé tel que revendiqué dans la revendication 10, caractérisé en ce que l'on fait réagir un oxirane de formule

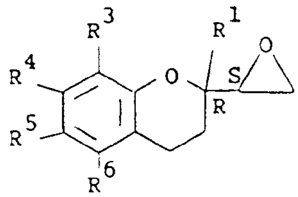

ou

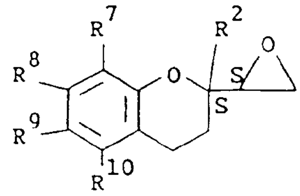

(II-a) (II-b)

avec une amine de formule

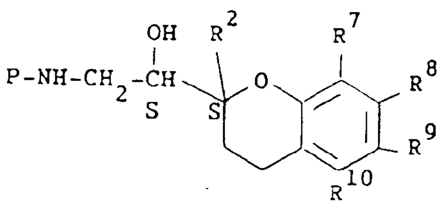

(III-a)

ou bien de formule

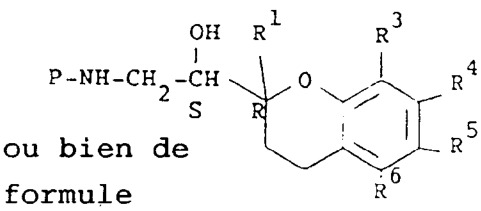

(III-b)

ou bien on fait réagir un réactif $PNH_2$ avec les composés (II-a) et (II-b) dans une technique à charge unique ; dans un solvant inerte dans les conditions de la réaction ; et dans lesquels P est de l'hydrogène ou un groupe protecteur N ; et où P est un groupe protecteur N, en déprotégeant les dérivés protégés en N ainsi obtenus de formule (I);

et, si on le souhaite, en préparant un sel d'addition d'acide pharmaceutiquement acceptable par traitement avec un acide ; ou à l'inverse, en préparant la forme de base libre par traitement au moyen d'une base.